# EUROPEAN PATENT APPLICATION

(11) **EP 3 960 175 A1**
(43) Date of publication of application: **02.03.2022**
(21) Application number: 20796243.2
(22) Date of filing: 24.04.2020
(51) Int. Cl.: A61K 31/445, A61K 31/454, A61P 9/10

(54) **USE OF COMPOUND IN PREPARATION OF DRUG FOR TREATING ATHEROSCLEROSIS**

(30) Priority: 26.04.2019 CN 201910347198
(71) Applicant: Generos Biopharma Ltd., Hangzhou, Zhejiang 310018 (CN)
(72) Inventor: FU, Xin-Yuan, Hangzhou, Zhejiang 310018 (CN); LIU, Xinyu, Hangzhou, Zhejiang 310018 (CN); ZHOU, Yi, Hangzhou, Zhejiang 310018 (CN); LUFEI, Chengchen, Hangzhou, Zhejiang 310018 (CN); LU, Cenbin, Hangzhou, Zhejiang 310018 (CN)
(74) Representative: Williams, Gareth Owen
(86) International application number: PCT/CN2020/086754
(87) International publication number: WO 2020/216335

(57) **Abstract**

Disclosed is use of a compound in the preparation of a medicament for treating atherosclerosis. The compound is selected from a group consisting of a compound of formula I, Nib2 and a pharmaceutically acceptable salt thereof. Based on the mechanism of atherogenesis, the invention started with the inhibition of inflammatory reaction. The research results showed that the compounds disclosed herein can significantly inhibit the macrophage foaming, reduce the deposition of lipidic necrotic substances, and reduce the formation of plaques, thus to some extent delaying or inhibiting atherosclerosis.

## Description

The present application claims priority to Chinese patent application No. 201910347198.3, filed April 26, 2019, the content of which is hereby incorporated by reference in its entirety.

### Technical Field

The present invention relates to the field of medicine, and in particular to the use of a compound in the preparation of a medicament for the treatment of atherosclerosis.

### Background

Atherosclerosis (AS) is the main cause of coronary heart disease, cerebral infarction and peripheral vascular diseases. Lipodystrophy is the pathological basis of atherosclerosis, characterized by the lesions of affected arteries starting from the intima. Generally, the accumulation of lipids and complex carbohydrates, bleeding and thrombosis occur first, then followed by hyperplasia of fibrous tissue and deposition of calcium, with gradual degeneration and calcification of the middle artery, resulting in the thickening and hardening of the arterial wall and the narrowing of the lumen of blood vessel. The pathological changes often involve large and medium-sized muscular arteries. Once they develop enough to block the arterial lumen, the tissue or organ supplied by the artery would be ischemic or necrotic. Since the appearance of lipids accumulated in the intima of the artery has yellow porridge look, it is called atherosclerosis.

Currently, atherosclerosis treatment mainly includes drug therapies and surgery. Drugs mainly include hypolipidemic drugs, such as statins, fibrates, nicotinic acid, cholestyramine, clofibrate, unsaturated fatty acids such as Yishouning^{™}, Xuezhiping^{™} and Xinmaile^{™}, etc., and alginic sodium diester; antiplatelet drugs, such as aspirin, dipyridamole, clopidogrel and cilostazol; vasodilator drugs, such as hydralazine (mainly acting on arteries), nitroglycerin and isosorbide dinitrate (mainly acting on veins), sodium nitroprusside (acting on both arteries and veins), α1 receptor blockers such as prazosin, α2 receptor blockers such as phentolamine, β2 receptor agonists such as salbutamol, captopril, enalapril, nifedipine, diltiazem, methionine, minoxidil, prostaglandin, atrial natriuretic peptide, and etc.; thrombolytic drugs such as urokinase, streptokinase, tissue-type plasminogen activator, single chain urokinase-type plasminogen activator, and TNK tissue-type plasminogen activator; and anticoagulant drugs such as heparin, enoxaparin, nadroparin and bivalirudin. Surgery includes recanalization, reconstruction or bypass grafting of narrow or occluded arteries and interventional therapy such as endovascular stent placement.

However, all of those drugs are used for symptomatic treatment and helpless regarding prevention of atherogenesis. Hypolipidemic drugs reduce the deposition of fatty substance in vessels by reducing blood lipid. Vasodilator drugs can be used as emergency drugs when blood pressure increases due to the formation of thrombosis and the hardening of blood vessel wall. Antiplatelet, thrombolytic and anticoagulant drugs to some extent can reduce the formation of thrombosis. Since these drugs function mainly through affecting metabolic activities in the body, long-term use will affect the normal physiological function of the body, leading to significant side effects. Further, these drugs cause great damage to liver and kidney function, and long-term use will inevitably lead to liver and kidney diseases. Therefore, there is an urgent need to find drugs that can effectively treat atherosclerosis.

### Summary

To solve the technical problems identified above, the present invention provides use of a compound in the preparation of a medicament for treatment of atherosclerosis. Based on the mechanism of atherogenesis, the present inventor started with the inhibition of inflammatory reaction. The research results of the present invention showed that the compounds of the present invention can significantly inhibit the macrophage foaming, reduce the deposition of lipidic necrotic substances, and reduce the formation of plaques, thus to some extent delaying or inhibiting atherosclerosis. The present invention addresses the formation of atherosclerosis from the origin, with less side effects and less damage to the functions of liver, kidney and other organs.

To solve the technical problems identified above, a first aspect of the present invention provides use of a compound in the preparation of a medicament for treatment of atherosclerosis, wherein the compound is selected from the group consisting of a compound of formula I, Nib2 and a pharmaceutically acceptable salt thereof; 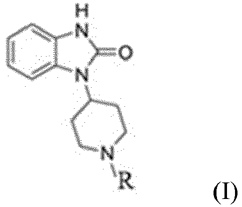
wherein, R is 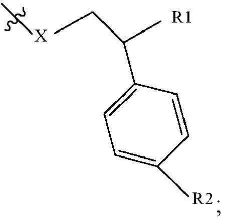
wherein, X is -CH2-CH2- or 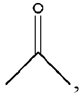
R1 is H or 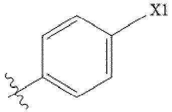 and X1 is a halogen;
R2 is CH3 or CX2, and X2 is a halogen;
wherein, Nib2 has a chemical formula of C₁₆H₁₁N₃O₃ and a structural formula of

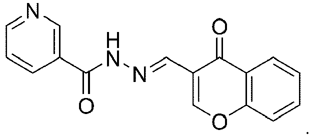

Preferably, the compound of formula I is Nib1, X7 or X8, wherein Nib1 has a chemical formula of C₂₈H₂₉F₂N₃O and a structural formula of 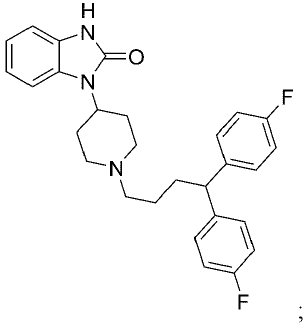
X7 has a chemical formula of C₂₁H₂₃N₃O₂ and a structural formula of 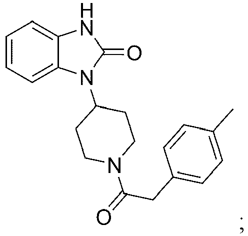 and
X8 has a chemical formula of C₂₁H₂₀F₃N₃O₂ and a structural formula of

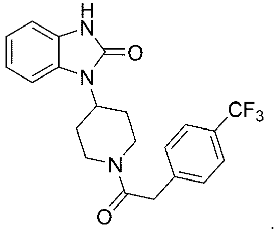

In a preferred embodiment of the invention, the invention provides use of Nib1 in the preparation of a medicament for treatment of atherosclerosis, wherein Nib1 has a chemical formula of C₂₈H₂₉F₂N₃O and a structural formula of

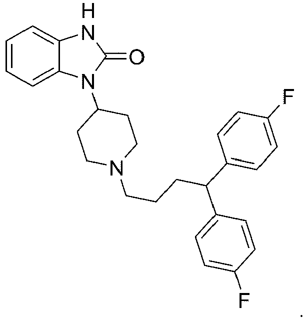

In a preferred embodiment of the invention, the invention provides use of Nib2 in the preparation of a medicament for treatment of atherosclerosis, wherein Nib2 has a chemical formula of C₁₆H₁₁N₃O₃ and a structural formula of

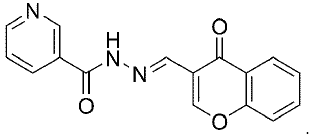

In a preferred embodiment of the invention, the invention provides use of X7 in the preparation of a medicament for treatment of atherosclerosis, wherein X7 has a chemical formula of C₂₁H₂₃N₃O₂ and a structural formula of

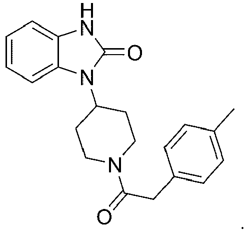

In a preferred embodiment of the invention, the invention provides use of X8 in the preparation of a medicament for treatment of atherosclerosis, wherein X8 has a chemical formula of C₂₁H₂₀F₃N₃O₂ and a structural formula of

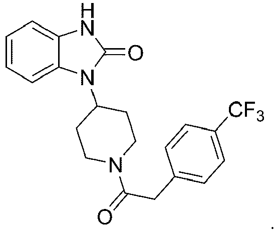

In the present disclosure, the activity of the compounds Nib1, Nib2, X7 and X8 in delaying or inhibiting atherosclerosis hinges on the present inventors' following observation. Atherosclerosis starts with the accumulation of low-density lipoprotein, which leads to dysfunction of endothelial cells, and then induces the disease along with other sclerosing factors. Activated vascular endothelial cells stimulate a series of chemokines and increase the expression of proteins that adhere to the cell surface. Monocytes differentiate into macrophages, while increasing the expression of pattern recognition receptors on their surface, taking modified low-density lipoproteins and promoting inflammation, resulting in foam cells that are full of lipid. The constant accumulation of modified low-density lipoproteins and the disturbed cell lipid homeostasis leads to necrosis of the foam cells, which, in turn, causes fat deposition and deterioration of inflammation. Smooth muscle cells transfer from vascular media to intima, stabilize plaque proliferation, absorb modified lipoproteins, and secrete cytoplasmic matrix proteins. Persistent inflammation occurs because cytokines destabilize plaques by reducing the production of cytoplasmic matrix proteins, increase the production/activity of extracellular matrix proteins that degrade matrix metalloproteinases, and reduce the expression/activity of these enzyme inhibitors. Plaque rupture leads to platelets aggregation, coagulation and thrombosis, and eventually leads to clinical complications of the disease. Cytokines can induce and regulate the expression or activity of key downstream genes in cell signaling, affect the interaction between immune and endothelial cells. By disrupting this homeostasis, formation of macrophage foam cells, and ultimately, vascular embolism, is regulated. In the present invention, Nib1/Nib2/X7/X8 can significantly inhibit the foaming of macrophages, reduce the formation of plaques, and have low toxic and side effects.

Preferably, compound Nib 1 is administered at a dosage of 0.1 to 1.098 mg/kg.

Preferably, compound Nib2 is administered at a dosage of 0.109 to 5 mg/kg.

Preferably, compound X7 and/or X8 is administered at a dosage of 0.1 to 5 mg/kg.

Preferably, compound Nib1 is administered at a dosage of 0.219 to 1.098 mg/kg, for example, 0.549 mg/kg.

Preferably, compound Nib2 is administered at a dosage of 0.109 to 0.549 mg/kg, for example, 0.219 mg/kg.

Preferably, compound X7 and/or X8 is administered at a dosage of 0.549 to 1.098 mg/kg.

Preferably, the compounds are present in the form of pharmaceutical compositions. The pharmaceutical compositions preferably comprise pharmaceutically acceptable carriers and/or excipients.

Preferably, the compounds are formulated into oral or injection formulations. The oral formulations are preferably capsules or tablets.

Preferably, the compounds can improve abnormal blood lipid metabolism, preferably reduce the levels of TC, HDL and/or LDL, significantly inhibit the foaming of macrophages, significantly reduce the deposition of macrophages in the atheroma, reduce the deposition of lipidic necrosis substances and/or decrease the formation of atherosclerotic plaques.

In order to solve the above technical problems, a second aspect of the present invention provides a drug for treatment of atherosclerosis, wherein the drug comprises a compound selected from a group consisting of a compound of formula I, Nib2 and a pharmaceutically acceptable salt thereof; 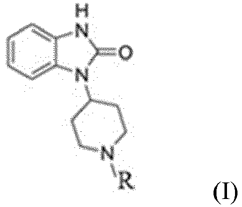
wherein, R is 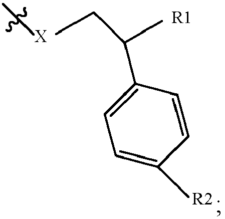
wherein, X is -CH2-CH2- or 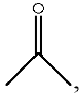
R1 is H or 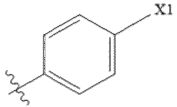 and X1 is a halogen;
R2 is CH3 or CX2, and X2 is a halogen;
wherein, Nib2 has a chemical formula of C₁₆H₁₁N₃O₃ and a structural formula of

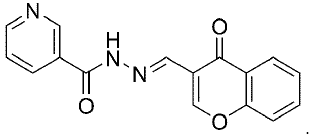

Preferably, the compound of formula I is Nib1, X7 or X8, wherein Nib1 has a chemical formula of C₂₈H₂₉F₂N₃O and a structural formula of 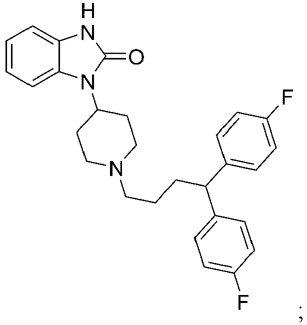
X7 has a chemical formula of C₂₁H₂₃N₃O₂ and a structural formula of 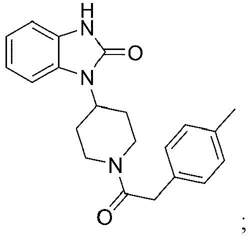 and
X8 has a chemical formula of C₂₁H₂₀F₃N₃O₂ and a structural formula of

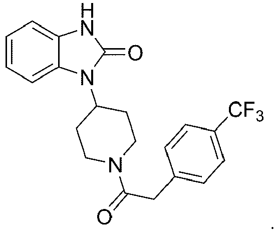

Preferably, compound Nib 1 is administered at a dosage of 0.1 to 1.098 mg/kg.

Preferably, compound Nib2 is administered at a dosage of 0.109 to 5 mg/kg.

Preferably, compound X7 and/or X8 is administered at a dosage of 0.1 to 5 mg/kg.

Preferably, compound Nib1 is administered at a dosage of 0.219 to 1.098 mg/kg, for example, 0.549 mg/kg.

Preferably, compound Nib2 is administered at a dosage of 0.109 to 0.549 mg/kg, for example, 0.219 mg/kg.

Preferably, compound X7 and/or X8 is administered at a dosage of 0.549 to 1.098 mg/kg.

Preferably, the drug comprises a pharmaceutically acceptable carrier and/or excipient.

Preferably, the drug is formulated into an oral or injection formulation. The oral formulation is preferably a capsule or a tablet.

Preferably, the drug can improve abnormal blood lipid metabolism, preferably reduce the levels of TC, HDL and/or LDL, significantly inhibit the foaming of macrophages, significantly reduce the deposition of macrophages in the atheroma, reduce the deposition of lipidic necrosis substances and/or decrease the formation of atherosclerotic plaques.

In order to solve the above technical problems, a third aspect of the present invention provides a compound as described in the first or second aspect of the present invention for use in the prevention and/or treatment of atherosclerosis.

In order to solve the above-mentioned technical problems, a fourth aspect of the present invention provides a method for preventing and/or treating atherosclerosis, the method comprising using a compound according to the first or second aspect of the present invention.

In order to solve the above-mentioned technical problems, a fifth aspect of the present invention provides a drug for improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques, wherein the drug comprises a compound according to the first or second aspect of the present invention.

In order to solve the above-mentioned technical problems, a sixth aspect of the present invention provides a method for improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques, wherein the method comprises using a compound according to the first or second aspect of the present invention.

In order to solve the above-mentioned technical problems, a seventh aspect of the present invention provides a compound according to the first or second aspect of the present invention for use in improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques.

In order to solve the above-mentioned technical problems, an eighth aspect of the present invention provides use of a compound according to the first or second aspect of the present invention in the preparation of a medicament for improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques.

In the present invention, the above-mentioned dosages for humans are calculated according to the dosage of the animal experiment of the present invention, and specifically is the mouse dosage divided by the conversion factor of 9.1. However, those skilled in the art should understand that the dosages within an generally accepted error range in the art should be also within the scope of the present invention, for example, the difference is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, and etc.

The present invention is advantageous over prior arts in that the compounds of the present invention were found effective in ApoE knockout mice to improve blood lipids. Atherosclerotic plaques can be obviously seen in the aorta of ApoE knockout mice. After feeding the compound of the present invention, the atherosclerotic plaques are significantly improved. Detection of blood lipid content related to atherosclerosis found that the compounds of the present invention can reduce the levels of TC, HDL, and LDL in the serum of mice, and can significantly reduce the deposition of macrophages in the atherosclerotic plaque. Both *in vitro* and *in vivo* studies showed that the compounds of the present invention can improve abnormal blood lipid metabolism, prevent and treat atherosclerosis. Compared with statins, the present invention can effectively prevent and treat atherosclerosis in experimental animals without increasing the incidence of diabetes, and does not damage liver and kidney functions.

### Brief Description of the Drawings

Figure 1 is a micrograph (HE x 100) of a frozen section of mouse 59# in model control group.
Figure 2 is a micrograph (oil red O x 100) of a frozen section of mouse 59# in model control group.
Figure 3 is a micrograph (HE x 100) of a frozen section of mouse 57# in statin treatment group.
Figure 4 is a micrograph (oil red O x 100) of a frozen section of mouse 57# in statin treatment group.
Figure 5 is a micrograph (HE x 100) of a frozen section of mouse 69# in sample A low dosage group.
Figure 6 is a micrograph (oil red O x 100) of a frozen section of mouse 69# in sample A low dosage group.
Figure 7 is a micrograph (HE x 100) of a frozen section of mouse 24# in sample A medium dosage group.
Figure 8 is a micrograph (oil red O x 100) of a frozen section of mouse 24# in sample A medium dosage group.
Figure 9 is a micrograph (HE x 100) of a frozen section of mouse 49# in sample A high dosage group.
Figure 10 is a micrograph (oil red O x 100) of a frozen section of mouse 49# in sample A high dosage group.
Figure 11 is a micrograph (HE x 100) of a frozen section of mouse 55# in sample B low dosage group.
Figure 12 is a micrograph (oil red O x 100) of a frozen section of mouse 55# in sample B low dosage group.
Figure 13 is a micrograph (HE x 100) of a frozen section of mouse 8# in sample B medium dosage group.
Figure 14 is a micrograph (oil red O x 100) of a frozen section of mouse 8# in sample B medium dosage group.
Figure 15 is a micrograph (HE x 100) of a frozen section of mouse 64# in sample B high dosage group.
Figure 16 is a micrograph (oil red O x 100) of a frozen section of mouse 64# in sample B high dosage group.
Figure 17 shows aorta (VCAM-1): blood vessels and plaques have low to medium signal expression (model control group 16#IHC-P×400).
Figure 18 shows aorta (VCAM-1): blood vessels have low to medium signal expression (statin treatment group 9#IHC-P×400).
Figure 19 shows aorta (VCAM-1): blood vessels have low to medium signal expression (sample A low dosage group 35#IHC-P×400).
Figure 20 shows aorta (VCAM-1): blood vessels have low to medium signal expression (sample A medium dosage group 50#H-IC-P×400).
Figure 21 shows aorta (VCAM-1): blood vessels have low to medium signal expression (sample A high dosage group 2#IHC-P×400).
Figure 22 shows aorta (VCAM-1): blood vessels have low to medium signal expression (sample B low dosage group 3#H-IC-P×400).
Figure 23 shows aorta (VCAM-1): blood vessels have low to medium signal expression (sample B medium dosage group 44#H-IC-P×400).
Figure 24 shows aorta (VCAM-1): blood vessels have low to medium signal expression (sample B high dosage group 23#H-IC-P×400).
Figure 25 shows aorta (ICAM-1): blood vessels and plaques have medium signal expression (model control group 1#IHC-P×400).
Figure 26 shows aorta (ICAM-1): blood vessels have low to medium signal expression (statin treatment group 30#IHC-P×400).
Figure 27 shows aorta (ICAM-1): blood vessels have low to medium signal expression (sample A low dosage group 65#IHC-P×400).
Figure 28 shows aorta (ICAM-1): blood vessels have low to medium signal expression (sample A medium dosage group 24#H-IC-P×400).
Figure 29 shows aorta (ICAM-1): blood vessels have low to medium signal expression (sample A high dosage group 48#IHC-P×400).
Figure 30 shows aorta (ICAM-1): blood vessels have low to medium signal expression (sample B low dosage group 25#H-IC-P×400).
Figure 31 shows aorta (ICAM-1): blood vessels have low to medium signal expression (sample B medium dosage group 10#H-IC-P×400).
Figure 32 shows aorta (ICAM-1): blood vessels have low to medium signal expression (sample B high dosage group 66#H-IC-P×400).
Figure 33 shows aorta (CD68): blood vessels and plaques have medium signal expression (model control group 29#IHC-P×400).
Figure 34 shows aorta (CD68): blood vessels have low to medium signal expression (statin treatment group 56#IHC-P×400).
Figure 35 shows aorta (CD68): blood vessels have low to medium signal expression (sample A low dosage group 62#IHC-P×400).
Figure 36 shows aorta (CD68): blood vessels have low to medium signal expression (sample A medium dosage group 24#H-IC-P×400).
Figure 37 shows aorta (CD68): blood vessels have low to medium signal expression (sample A high dosage group 19#IHC-P×400).
Figure 38 shows aorta (CD68): blood vessels have low to medium signal expression (sample B low dosage group 15#H-IC-P×400).
Figure 39 shows aorta (CD68): blood vessels have low to medium signal expression (sample B medium dosage group 43#H-IC-P×400).
Figure 40 shows aorta (CD68): blood vessels have low to medium signal expression (sample B high dosage group 39#H-IC-P×400).
Figure 41 is a histogram made according to the results in Table 9, comparing the pathological results of test samples against atherosclerotic lesions in ApoE knockout mice.
Figure 42 is a graph of body weight changes in compound Nib1 treatment group, compared with healthy controls, vehicle and atorvastatin treatment groups.
Figure 43 is a graph of body weight changes in compound Nib2 (N2) treatment group, compared with healthy controls, vehicle and atorvastatin treatment groups.
Figure 44 is a graph of body weight changes in compound X7 treatment group, compared with healthy controls, vehicle and atorvastatin treatment groups.
Figure 45 is a graph of body weight changes in compound X8 treatment group, compared with healthy controls, vehicle and atorvastatin treatment groups.
Figure 46 is a histogram of the plaque area ratio of the inner wall of the aorta in each treatment group; A is the plaque ratio of the entire arterial arch (longitudinal section, whole); B is the cross-sectional view of the arterial outflow tract for analyzing the plaque ratio.
Figure 47 shows the blood lipid (TG, TCHO, HDL-C, LDL-C) levels of each group of animals before the start of the experiment.
Figure 48 shows the animal blood lipid (TG, TCHO, HDL-C, LDL-C) levels at the end of the experiment.
Figure 49 shows the effect of Nib1 treatment on blood lipids (TG, TCHO, HDL-C, LDL-C).
Figure 50 shows the effect of Nib2 treatment on blood lipids (TG, TCHO, HDL-C, LDL-C).
Figure 51 shows the effect of X7 treatment on blood lipids (TG, TCHO, HDL-C, LDL-C).
Figure 52 shows the effect of X8 treatment on blood lipids (TG, TCHO, HDL-C, LDL-C).

### Detailed Description of Embodiments

The present invention will be described in detail in reference to the examples.

### Example 1. Studies of the effects of samples A (Nib1) and B (Nib2) on atherogenesis in ApoE knockout mice.

Reagents: Nib1 was purchased from Sigma (Lot # P1793), and Nib2 from EMD Millipore Corporation (Lot # 573108). Atorvastatin calcium tablets were produced by Beijing Jialin Pharmaceutical Co., Ltd., Lot # MC16035. Sodium carboxymethyl cellulose (CMC-Na) was purchased from Tianjin Fuchen Chemical Reagent Factory (Lot # 20170220); DMSO was purchased from Guangdong Guanghua Technology Co., Ltd. (Lot # 20170215); polyethylene glycol PEG300 was purchased from Shanghai Macklin Biochemical Technology Co., Ltd. (Lot # C10113353).

**Table 1. Antibodies used in immunohistochemical assays**

| Name | Lot # | Exp. Date | Manufacturer |
|---|---|---|---|
| Multimer anti-rabbit/mouse IgG-HRP | 13G27D604 | 201907 | Wuhan Boster Biological Engineering Co., Ltd. |
| Primary anti-ICAM-1 | E0117 | 20190803 | SANTA CRUZ BIOTECHNOLOGY,INC. |
| Primary anti-VCAM-1 | G3014 | 20190803 | SANTA CRUZ BIOTECHNOLOGY,INC. |
| Primary anti-CD68 | D1117 | 20190803 | SANTA CRUZ BIOTECHNOLOGY,INC. |

Methods: SPF grade C57BL/6 ApoE -/-model mice were provided by Guangdong Medical Experimental Animal Center, laboratory animal production license No. SCXK (Guangdong) 2013-0002. Laboratory animal quality certificate number: 44007200048196). Mice were half male and half female, aged 8 to 12 weeks, and 72 animals in total. After four weeks of housing in a SPF-level animal facility, animals with less body weights were disposed. A total of 64 animals were randomly divided into 8 groups, 8 in each group, fed with 10% high-fat food, and received with different treatments. Animals were observed and administered with the medicines by gavage once daily. The body weights were measured once a week and continued for 58 days. Atorvastatin calcium tablets (abbreviated as statins) were ground and prepared with 0.5% sodium carboxymethyl cellulose solution. The test sample was dissolved in dimethyl sulfoxide solution and prepared with 30% PEG300. The grouping was shown in Table 2 below.

**Table 2. Dosages and Groups**

| Groups | Animals (n) | Dosages (mg/kg body weight) | Routes and Frequencies | Con. (mg/mL) |
|---|---|---|---|---|
| Model Control Group | 8 | - | gavage, QD | solvent |
| Statin Treatment Group | 8 | 20 | gavage, QD | 2.0 |
| Sample A Low Dosage | 8 | 2 | gavage, QD | 0.2 |
| Sample A Medium Dosage | 8 | 5 | gavage, QD | 0.5 |
| Sample A High Dosage | 8 | 10 | gavage, QD | 1.0 |
| Sample B Low Dosage | 8 | 1 | gavage, QD | 0.1 |
| Sample B Medium Dosage | 8 | 2 | gavage, QD | 0.2 |
| Sample B High Dosage | 8 | 5 | gavage, QD | 0.5 |

At the end of the experiment, animals were anesthetized with pentobarbital sodium. Blood was collected from the orbital venous plexus, and centrifuged at 3000 r/min at a low temperature for 10 min to separate the serum. The levels of total cholesterol TC, triglyceride TG, high-density lipoprotein HDL-C, and low-density lipoprotein LDL-C were measured, and the remaining serum was stored in a refrigerator at -80 °C.

After blood collection, the animals were sacrificed, and the aortic arch together with the heart and thoracic aorta were fixed with neutral formaldehyde and subject to pathological examination. The abdominal aorta was collected and quick-frozen in liquid nitrogen, and stored at -80 °C.
a) HE staining: aortic arch was sliced and HE stained. The plaques were observed under optical microscope.
b) Oil red O staining: aortic arch was sliced and oil red O stained. The plaques were observed under optical microscope.
c) Immunohistochemistry: The thoracic aorta was sliced and detected by immunohistochemistry for the expression of CD68, intercellular adhesion molecule-1 (ICAM-1), and vascular cell adhesion Molecule-1 (VCAM-1).

Statistical analysis: Data were expressed as the mean plus or minus the standard deviation (*̅x̅*̅±̅*s*), and analyzed by SPSS21.0 statistical software. The weight data of each group were compared by repeated measures analysis of variance; the blood lipid levels of the statin treatment group were compared with the model control group by independent sample T test; the blood lipid levels of the test samples A and B were compared with the model control group by one-way analysis of variance, and LSD method was used for the comparison among groups. The level of test is α=0.05.

### Results

General observation: No obvious abnormalities were seen during the experiment.

Body weights (Tables 3-5): Compared with the model control group, there was no statistical difference in the body weight of each group of mice at each measurement time (P>0.05), indicating that its toxic and side effects were relatively small.

**Table 3. The effect of test samples on the body weight of ApoE knockout mice (x̅+s, g)**

| Groups | *n* | d1 | | d8 | | d15 | | d22 | | d29 | |
|---|---|---|---|---|---|---|---|---|---|---|---|
| | | ♂ | ♀ | ♂ | ♀ | ♂ | ♀ | ♂ | ♀ | ♂ | ♀ |
| Model Control | 4 | 29.6 ±17 | 21.9 ±1.7 | 29.2 ±1.9 | 22.7 ±1.7 | 29.3 ±2.0 | 22.6 ±1.6 | 29.4 ±1.8 | 23.1± 1.4 | 29.4 ±1.9 | 23.2 ±2.0 |
| Statin Treatment | 4 | 28.9 ±12 | 21.2 ±0.9 | 28.9 ±0.9 | 22.0 ±0.6 | 29.0 ±1.1 | 21.5 ±0.7 | 29.0 ±1.4 | 21.4± 0.6 | 29.1 ±1.2 | 21.4 ±0.6 |
| Sample A Low Dosage | 4 | 28.9 ±1.3 | 21.2 ±0.9 | 27.8 ±1.0 | 22.2 ±1.0 | 28.1 ±1.0 | 22.3 ±0.9 | 28.0 ±1.0 | 23.1± 0.9 | 27.8 ±1.0 | 22.5 ±1.7 |
| Sample A Medium Dosage | 4 | 29.1 ±2.5 | 21.4 ±1.3 | 28.5 ±2.4 | 23.0 ±09 | 28.4 ±2.4 | 23.2 ±08 | 28.0 ±2.4 | 23.1± 1.2 | 28.1 ±2.2 | 23.4 ±0.7 |
| Sample A High Dosage | 4 | 29.0 ±1.0 | 21.2 ±0.9 | 30.0 ±0.9 | 22.4 ±1.4 | 29.7 ±1.2 | 22.7 ±0.8 | 29.2 ±1.7 | 21.4± 0.9 | 28.3 ±1.3 | 23.0 ±0.6 |
| Sample B Low Dosage | 4 | 28.9 ±0.9 | 21.1 ±0.7 | 28.4 ±1.4 | 21.8 ±0.7 | 28.1 ±1.3 | 22.1 ±1.0 | 28.0 ±1.2 | 21.8± 1.0 | 28.1 ±1.5 | 22.2 ±0.9 |
| Sample B Medium Dosage | 4 | 29.0 ±2.5 | 21.7 ±1.9 | 28.8 ±2.5 | 22.1 ±2.3 | 28.9 ±2.3 | 22.1 ±16 | 28.7 ±2.4 | 22.2± 2.0 | 29.0 ±2.4 | 22.8 ±2.3 |
| Sample B High Dosage | 4 | 29.2 ±1.9 | 21.5 ±1.2 | 29.2 ±2.0 | 22.5 ±1.0 | 29.0 ±1.8 | 22.3 ±1.2 | 29.2 ±2.1 | 22.3± 1.3 | 29.4 ±2.2 | 22.3 ±1.3 |

| | | | | | | | | | | | |
|---|---|---|---|---|---|---|---|---|---|---|---|
| Note: repeated measures analysis of variance | | | | | | | | | | | |

**Table 4. The effect of test samples on the body weight of ApoE knockout mice (x̅+s, g)**

| Groups | *n* | d36 | | d43 | | d50 | | d57 | | d58 | |
|---|---|---|---|---|---|---|---|---|---|---|---|
| | | ♂ | ♀ | ♂ | ♀ | ♂ | ♀ | ♂ | ♀ | ♂ | ♀ |
| Model Control | 4 | 29.4 ±18 | 23.4 ±2.5 | 29.5 ±1.7 | 23.0 ±1.6 | 30.0 ±1.8 | 23.8 ±1.6 | 30.6 ±1.8 | 24.2± 1.9 | 30.3± 1.5 | 23.6± 1.9 |
| Statin Treatment | 4 | 29.2 ±1.0 | 22.2 ±0.9 | 29.6 ±0.8 | 22.4 ±0.5 | 29.8 ±0.6 | 23.1 ±0.9 | 30.0 ±1.6 | 22.7± 0.8 | 29.7± 1.4 | 22.8± 0.5 |
| Sample A Low Dosage | 4 | 27.9 ±1.1 | 22.8 ±1.2 | 28.2 ±1.3 | 23.5 ±1.2 | 28.4 ±1.1 | 23.9 ±0.6 | 28.3 ±0.6 | 24.8± 1.3 | 28.4± 0.8 | 25.0± 1.3 |
| Sample A Medium Dosage | 4 | 28.2 ±2.2 | 23.2 ±1.2 | 28.2 ±2.4 | 23.7 ±0.7 | 28.8 ±2.2 | 23.9 ±1.0 | 28.6 ±2.2 | 24.9± 0.6 | 28.8± 2.3 | 24.3± 0.6 |
| Sample A High Dosage | 4 | 28.8 ±1.7 | 22.6 ±0.7 | 28.6 ±1.5 | 23.0 ±1.3 | 28.8 ±1.1 | 22.8 ±0.6 | 29.4 ±1.3 | 24.3± 0.9 | 29.7± 1.5 | 23.6± 1.1 |
| Sample B Low Dosage | 4 | 28.0 ±1.7 | 22.9 ±1.0 | 27.9 ±1.7 | 23.4 ±1.4 | 28.4 ±1.9 | 22.8 ±1.4 | 28.2 ±2.6 | 23.0± 1.4 | 28.3± 2.3 | 22.8± 1.3 |
| Sample B Medium Dosage | 4 | 28.8 ±2.2 | 22.8 ±2.2 | 29.4 ±2.5 | 23.1 ±2.2 | 29.9 ±3.0 | 23.4 ±2.4 | 30.0 ±3.4 | 23.5± 2.0 | 30.2± 3.1 | 23.7± 2.3 |
| Sample B High Dosage | 4 | 29.3 ±2.3 | 22.5 ±1.2 | 29.9 ±2.7 | 23.3 ±1.4 | 29.8 ±2.8 | 23.4 ±1.5 | 29.6 ±3.5 | 22.9± 1.1 | 29.7± 3.2 | 23.1± 1.0 |

| | | | | | | | | | | | |
|---|---|---|---|---|---|---|---|---|---|---|---|
| Note: repeated measures analysis of variance | | | | | | | | | | | |

**Table 5. The effect of test samples on the body weight of ApoE knockout mice (x̅±s, g, half male and female)**

| Groups | *n* | d1 | d8 | d15 | d22 | d29 | d36 | d43 | d50 | d57 | d58 |
|---|---|---|---|---|---|---|---|---|---|---|---|
| Model Control | 8 | 25.7 ±4.4 | 25.9 ±3.9 | 25.9 ±3.9 | 26.3 ±3.7 | 26.3 ±3.8 | 26.4 ±3.8 | 26.2 ±3.8 | 26.9 ±3.7 | 27.4 ±3.8 | 27.0 ±3.9 |
| Statin Treatment | 8 | 25.1 ±4.2 | 25.4 ±3.8 | 25.3 ±4.1 | 25.2 ±4.2 | 25.2 ±4.2 | 25.7 ±3.8 | 26.0 ±3.9 | 26.5 ±3.7 | 26.4 ±4.1 | 26.2 ±3.8 |
| Sample A Low Dosage | 8 | 25.0 ±43 | 25.0 ±3.1 | 25.2 ±32 | 25.5 ±2.8 | 25.1 ±3.1 | 25.4 ±29 | 25.8 ±2.8 | 26.1 ±2.5 | 26.5 ±2.1 | 26.7 ±2.1 |
| Sample A Medium Dosage | 8 | 25.2 ±45 | 25.8 ±3.3 | 25.8 ±32 | 25.6 ±3.1 | 25.7 ±29 | 25.7 ±3.1 | 25.9 ±29 | 26.4 ±3.0 | 26.8 ±2.5 | 26.5 ±29 |
| Sample A High Dosage | 8 | 25.1 ±43 | 26.2 ±42 | 26.2 ±3.9 | 25.3 ±43 | 25.6 ±3.0 | 25.7 ±3.5 | 25.8 ±3.3 | 25.8 ±3.3 | 26.8 ±2.9 | 26.6 ±3.5 |
| Sample B Low Dosage | 8 | 25.0 ±42 | 25.1 ±3.7 | 25.1 ±3.3 | 24.9 ±3.5 | 25.1 ±3.4 | 25.4 ±3.0 | 25.7 ±2.8 | 25.6 ±3.4 | 25.6 ±3.4 | 25.6 ±3.4 |
| Sample B Medium Dosage | 8 | 25.3±4.4 | 25.5±42 | 25.5±4.1 | 25.5±40 | 25.9±4.0 | 25.8±3.8 | 26.2±4.0 | 26.7±43 | 26.7±4.4 | 27.0±43 |
| Sample B High Dosage | 8 | 25.4±4.4 | 25.8±3.9 | 25.6±3.8 | 25.7±40 | 25.8±4.1 | 25.9±4.0 | 26.6±40 | 26.6±4.0 | 26.3±43 | 26.4±4.1 |

| | | | | | | | | | | | |
|---|---|---|---|---|---|---|---|---|---|---|---|
| Note: repeated measures analysis of variance | | | | | | | | | | | |

### Analysis of blood lipids in disease model animals

**Table 6. The effects of test samples on four different blood lipids in ApoE knockout mice (x̅±s, mmol/L).**

| Groups | *n* | TC | | TG | | HDL-C | | LDL-C | |
|---|---|---|---|---|---|---|---|---|---|
| | | ♂ | ♀ | ♂ | ♀ | ♂ | ♀ | ♂ | ♀ |
| Model Control | 4 | 24.51±2.56 | 17.05±1.49 | 1.70±0.22 | 0.83±0.19 | 2.75±0.16 | 1.62±0.13 | 8.29±0.96 | 5.07±0.66 |
| Statin Treatment | 4 | 19.45±2.37* | 16.85±1.76 | 0.78±0.27** | 0.65±0.06 | 1.92±0.62* | 1.44±0.09 | 6.17±1.46 | 5.03±0.57 |
| Sample A Low Dosage | 4 | 25.84±3.63 | 13.88±1.77** | 1.51±0.92 | 0.86±0.16 | 2.26±0.54 | 1.34±0.20* | 8.60±1.79 | 3.86±0.95* |
| Sample A Medium Dosage | 4 | 26.34±2.50 | 15.41±1.32 | 0.94±0.15 | 0.70±0.11 | 1.83±0.24 | 1.42±0.09 | 7.87±1.38 | 4.30±0.39 |
| Sample A High Dosage | 4 | 25.64±1.32 | 14.07±0.64* | 1.02±0.22 | 0.76±0.07 | 1.92±0.18 | 1.33±0.15* | 7.78±0.40 | 3.86±0.38* |
| Sample B Low Dosage | 4 | 23.63±5.16 | 15.50±2.01 | 1.26±0.89 | 0.57±0.08 | 2.18±0.75 | 1.32±0.18* | 8.07±2.56 | 4.32±0.59 |
| Sample B Medium Dosage | 4 | 22.54±2.88 | 15.49±2.98 | 1.05±0.25 | 0.58±0.10 | 2.00±0.23* | 1.36±0.16* | 7.03±1.41 | 4.40±0.96 |
| Sample B High Dosage | 4 | 23.32±5.72 | 16.21±1.98 | 0.83±0.46* | 0.65±0.24 | 2.04±0.42* | 1.38±0.08* | 7.31±2.41 | 4.61±0.56 |

| | | | | | | | | | |
|---|---|---|---|---|---|---|---|---|---|
| Note: The statin treatment group was compared with the model control group by independent sample T test; the blood lipid levels of the test samples A and B were compared with the model control group by one-way analysis of variance, and LSD was used for the comparison among groups; *P<0.05, ** P<0.01. | | | | | | | | | |

**Table 7. The effects of the tested samples on the four blood lipids of ApoE knockout mice (x±s, mmol/L, half male and female).**

| Groups | Dosage (mg/kg body weight) | *n* | TC | TG | HDL-C | LDL-C |
|---|---|---|---|---|---|---|
| Model Control | / | 8 | 20.78±4.43 | 1.26±0.51 | 2.19±0.62 | 6.68±1.88 |
| Statin Treatment | 20 | 8 | 18.15±2.38 | 0.71±0.19* | 1.68±0.49 | 5.60±1.19 |
| Sample A Low Dosage | 2 | 8 | 1986±692 | 1.18±0.70 | 1.80±0.62 | 6.23±2.86 |
| Sample A Medium Dosage | 5 | 8 | 20.87±6.13 | 0.82±0.17 | 1.63±0.28* | 6.09±2.12 |
| Sample Dosage High | 10 | 8 | 19.85±6.26 | 0.89±0.20 | 1.62±0.35* | 5.82±2.13 |
| Sample BLow Dosage | 1 | 8 | 19.56±5.66 | 0.91±0.69 | 1.75±0.69 | 6.19±2.64 |
| Sample B Medium Dosage | 2 | 8 | 19.01±4.64 | 0.81±0.30 | 1.68±0.39 | 5.71±1.80 |
| Sample Dosage B High | 5 | 8 | 19.76±5.49 | 0.74±0.35* | 1.71±0.45 | 5.96±2.17 |

| | | | | | | |
|---|---|---|---|---|---|---|
| Note: The statin treatment group was compared with the model control group by independent sample T test; the blood lipid levels of the test samples A and B were compared with the model control group by one-way analysis of variance, and LSD was used for the comparison among groups; *P<0.05, ** P<0.01. | | | | | | |

♂, n=4: Compared with the model control group, the serum TC, TG, HDL-C levels of the mice in the statin treatment group were lower, with statistical differences (P<0.01 or 0.05); the TG level of the test sample B high-dosage group was lower, and the levels of HDL-C in the medium and high-dosage groups of the test sample B were lower, with a statistical difference (P<0.05).

♀, n=4: Compared with the model control group, the serum TC, TG, and HDL-C levels of the mice in the statin treatment group were reduced to varying degrees, but there was no statistical difference (P>0.05); the levels of TC, HDL, and LDL in the low and high-dosage group were lower with statistical differences (P<0.01 or 0.05); and the levels of HDL-C in the low, medium, and high-dosge groups of the test sample B were lower, with statistical differences (P<0.05).

Total, n=8: Compared with the model control group, the serum TG level of the mice in the statin treatment group was lower, with statistical difference (P<0.05); the medium and high-dosage groups of the test sample A had lower HDL-C levels, with statistical difference (P<0.05); the TG level of the high-dosage group of the test sample B was lower, with statistical difference (P<0.05).

From the above results, it can be seen that the test samples A and B have different degrees of regulation on blood lipid levels, and have the effect of improving blood lipids.

Pathological results of aortic HE and oil red O staining: The aortic histology scores of the sample A medium-dosage group and the sample B medium-dosage group were lower than the model control group, and the results were statistically different (P<0.05). The scores of the other groups were lower than or equal to the model control group, and there was no statistical difference (P>0.05).

**Table 8. Scores of lesions of aortic atherosclerosis**

| Description of Lesions | Lesions and Scores |
|---|---|
| No atherosclerotic plaque | No, 0 |
| Small amount of lipid-containing necrotic material deposits seen in the subintimal layer of the aorta | Mild, 1 |
| Fatty necrotic material deposits seen on the wall of the aorta | Moderate, 2 |
| Fatty necrotic material deposition, foam cells, plaques protruding into the lumen | Severe, 3 |

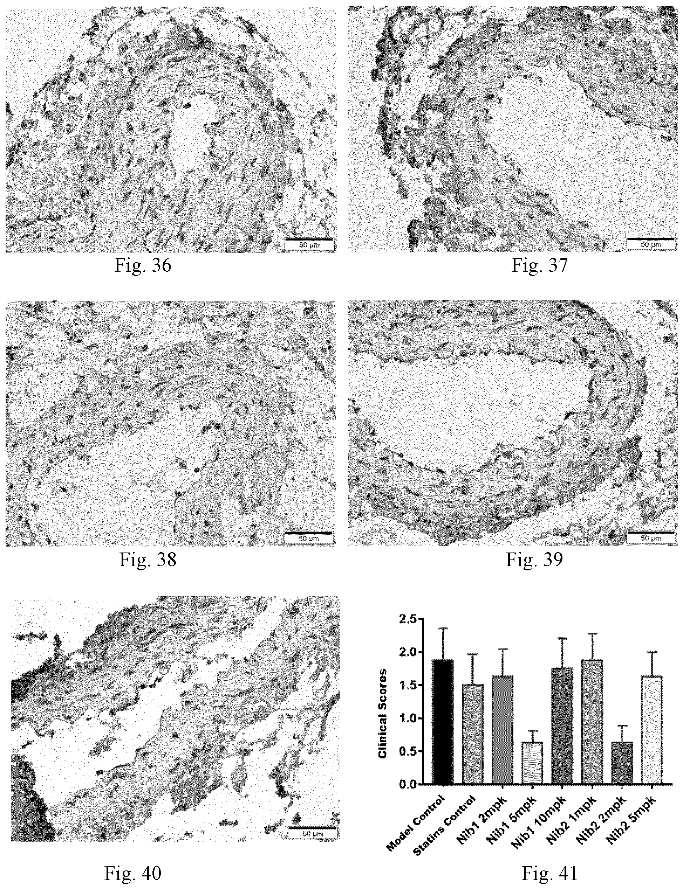

**Table 9. Pathological results of test samples on atherosclerotic lesions in ApoE knockout mice (see Figure 41 for histogram).**

| Groups | Dosage (mg/kg body weight) | Total *n* | Severe *n* | Moderate *n* | Mild *n* | No lesion *n* | Scores |
|---|---|---|---|---|---|---|---|
| Model Control | / | 8 | 4 | 1 | 1 | 2 | 1.88±1.36 |
| Statin Treatment | 20 | 8 | 2 | 3 | 0 | 3 | 1.50±1.31 |
| Sample A Low Dosage | 2 | 8 | 3 | 0 | 4 | 1 | 1.62±1.19 |
| Sample A Medium Dosage | 5 | 8 | 0 | 0 | 5 | 3 | 0.62±0.52* |
| Sample A High Dosage | 10 | 8 | 3 | 2 | 1 | 2 | 1.75±1.28 |
| Sample B Low Dosage | 1 | 8 | 3 | 2 | 2 | 1 | 1.88±1.13 |
| Sample B Medium Dosage | 2 | 8 | 0 | 1 | 3 | 4 | 0.62±0.74* |
| Sample B High Dosage | 5 | 8 | 2 | 2 | 3 | 1 | 1.62±1.06 |

| | | | | | | | |
|---|---|---|---|---|---|---|---|
| Note: Severe, moderate, mild, and no lesions were indicated as the number of animal cases, and the lesion score was the average score of the group. Severe lesions were defined as lipid-containing necrotic material deposits seen in the matrix of the aortic wall, and foam cells and plaques protruding into the lumen; moderate lesions were defined as lipid-containing necrotic material deposits seen in the aortic wall, and mild lesions were defined as small amount of fat-containing necrotic material deposition seen in the intima of the aorta. Single-factor analysis of variance was used for lesion score, * means P<0.05 compared with the model control group. | | | | | | | |

The results are described in detail as follows.

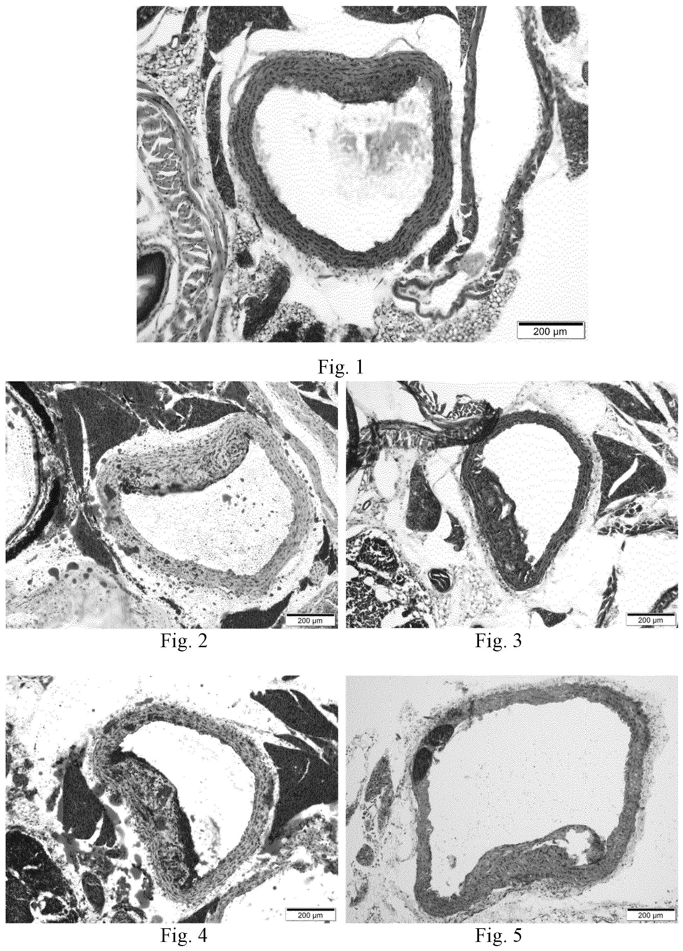

Model control group: 8 cases of animal aorta were submitted for examination. Histological observation showed that a total of 6 cases of animals had atherosclerosis, 4 of 6 (7#, 29#, 58#, 59#, as shown in Figures 1 and 2) were seen the deposition of lipid-containing necrotic material in the matrix of the aortic wall, and foam cells and plaques protruding toward the lumen, 1 animal (1#) was seen the deposition of lipid-containing necrotic material on the aortic wall, and 1 animal (52#) was seen a small amount of lipid-containing necrotic material deposition in the subintima of the aorta.

Statin treatment group: 8 cases of animal aorta were submitted for examination. Histological observation showed that a total of 5 cases of animals had atherosclerosis: 2 cases of animals (45#, 57#, as shown in Figures 3 and 4) were seen lipid-containing necrotic material deposits in the matrix of the vessel wall, and foam cells and plaques protruding to the lumen. 3 cases of animals (9#, 18#, 30#) were seen lipid-containing necrotic material deposits on the aortic wall.

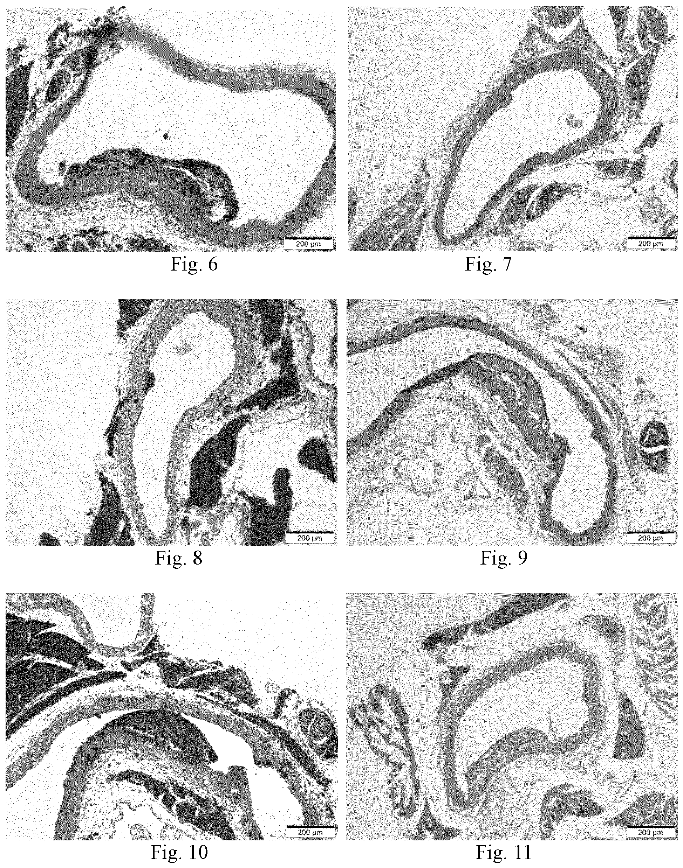

Sample A low dosage group: 8 cases of animal aorta were submitted for examination. Histological observation showed that a total of 7 cases of animals had atherosclerosis: 3 cases of animals (14#, 33#, 69#, as shown in Figures 5 and 6) were seen the deposition of lipid-containing necrotic material in the matrix of the aortic wall, and foam cells and plaques protruding toward the lumen. In 4 animals (35#, 62#, 65#, 68#) was seen a small amount of lipid-containing necrotic substance deposition in the aortic subintima.

Sample A medium dosage group: 8 cases of animal aorta were submitted for examination. Histological observation showed that a total of 5 cases of animals had atherosclerosis: 5 cases of animals (21#, 24# (as shown in Figures 7 and 8), 32 #, 61#, 70#) were seen a small amount of lipid-containing necrotic material deposited in the subintima of the aorta.

Sample A high dosage group: 8 cases of animal aorta were submitted for examination. Histological observation showed that a total of 6 cases of animals had atherosclerosis: 3 cases of animals (17#, 48#, 49#, as shown in Figures 9 and 10)) were seen the deposition of lipid-containing necrotic material in the matrix of the aortic wall, and foam cells and plaques protruding to the lumen, 2 cases of animals (26#, 63#) were seen the deposition of lipid-containing necrotic material on the aortic wall, and 1 case animal (41#) was seen a small amount of lipid-containing necrotic material deposited in the subintima of the aorta.

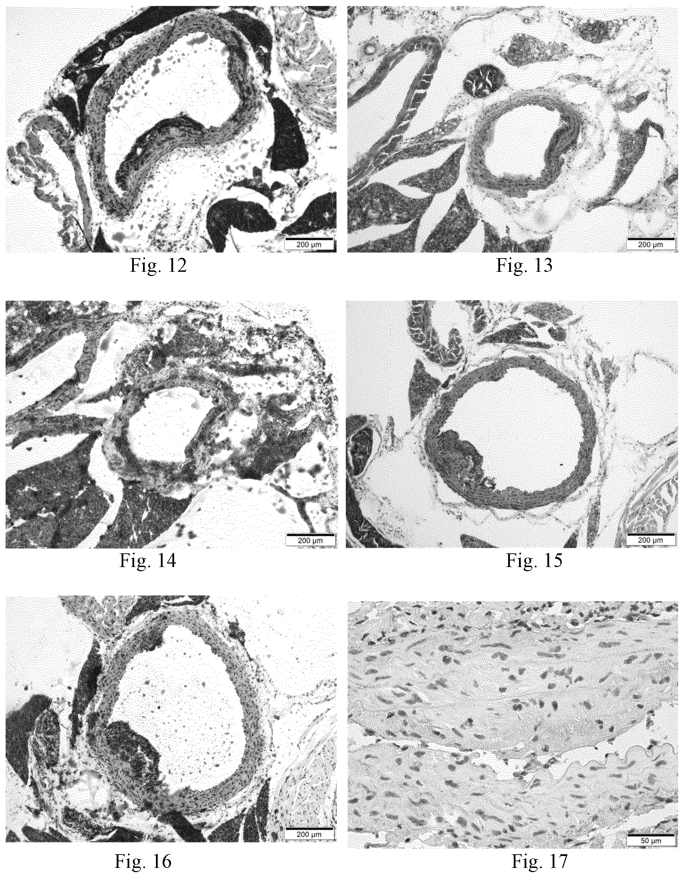

Sample B low dosage group: 8 cases of animal aorta were submitted for examination. Histological observation showed that a total of 7 cases of animals had atherosclerosis: 3 cases of animals (6#, 25#, 55# as shown in Figures 11 and 12) were seem the deposition of lipid-containing necrotic material in the matrix of the aortic wall, and foam cells and plaques protruding to the lumen, 2 cases of animals (15#, 60#) were seen the deposition of lipid-containing necrotic material on the aortic wall and 2 cases of animals (3#, 42#) was seen a small amount of lipid-containing necrotic material deposited in the subintima of the aorta.

Sample B medium dosage group: 8 cases of animal aorta were submitted for examination. Histological observation showed that a total of 4 cases of animals developed atherosclerosis: 1 case of animal (8# shown in Figures 13 and 14) was seen the deposition of lipid-containing necrotic material in the wall of aorta, and 3 cases of animals (40#, 43#, 44#) were seen a small amount of lipid-containing necrotic material deposited in the subintima of the aorta.

Sample B high dosage group: 8 cases of animal aorta were submitted for examination. Histological observation showed that a total of 7 cases of animals had atherosclerosis: 2 cases of animals (39#, 64#, as shown in Figures 15 and 16) were seen lipid-containing necrotic material deposited in the matrix of the aortic wall, and foam cells and plaque protruding to the lumen, 2 cases of animals (27#, 54#) were seen lipid-containing necrotic material deposited in the aortic wall, and 3 animals (11 #, 12#, 23#) were seen a small amount of lipid-containing necrotic material deposited in the subintima of the aorta.

It can be seen from the above results that the effect of samples A and B is equivalent to or better than that of the statin group; both can significantly inhibit the foaming of macrophages, reduce the deposition of lipid necrotic substances, reduce plaque formation, and to a certain extent delay or inhibit atherosclerosis.

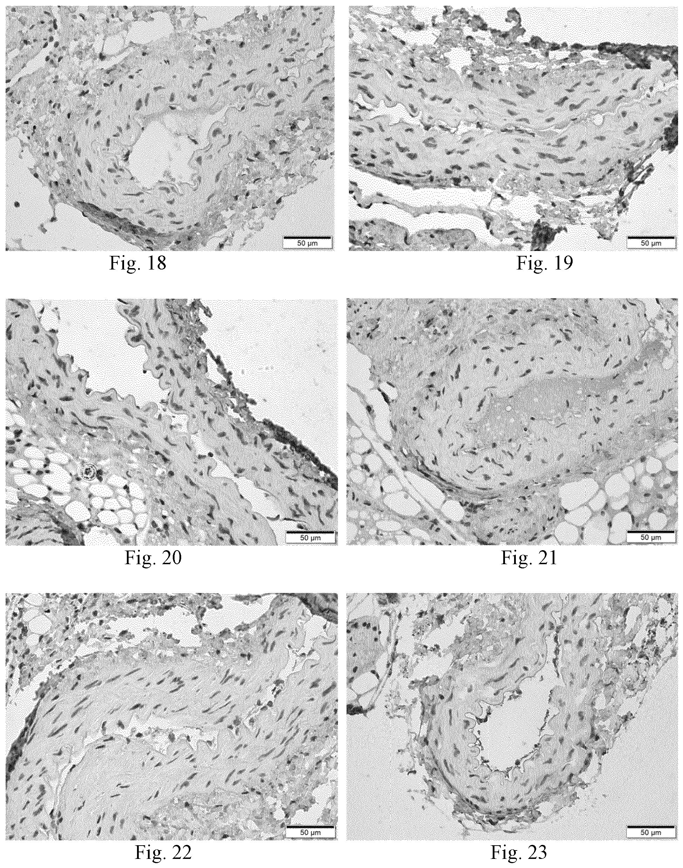

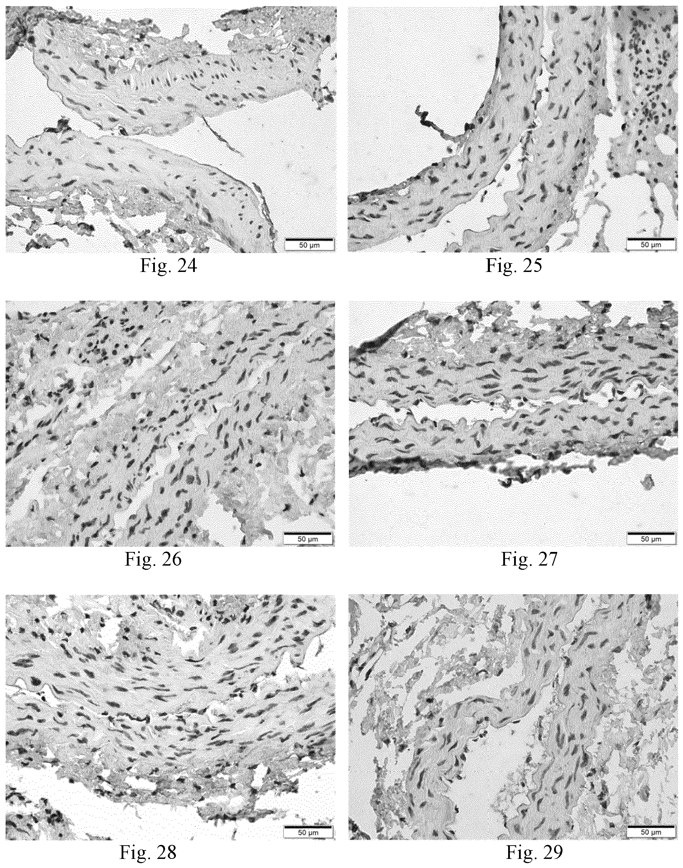

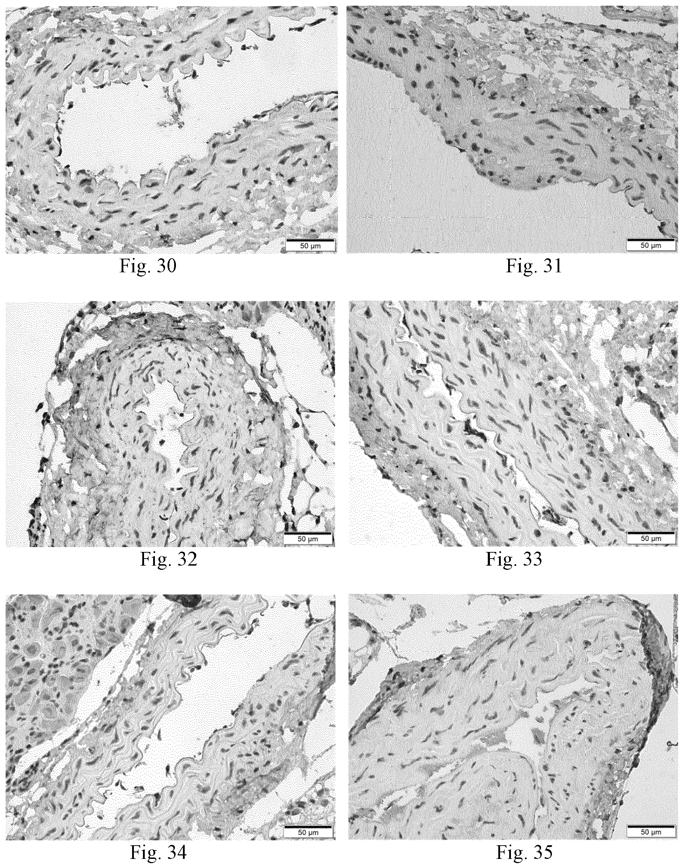

The results of immunohistochemistry are shown in Figures 17 to 40.
VCAM-1: The results of sample A in the medium and high dosage groups were lower than the model control group, with statistical differences (P<0.05);
ICAM-1: The results of the statin treatment group, the sample A low, medium, and high dosage groups, and the sample B medium dosage group were lower than the model control group, with statistical differences (P<0.05);

The results of CD68 were not statistically different compared to the model control group (P>0.05).

**Tabel 10. Results of semi-quantitative average optical density of VCAM-1, ICAM-1 and CD68 in the aortic wall of ApoE knockout mice by the test samples (x̅±s, x 10⁻²)**

| Groups | n | VCAM-1 average optical density (× 10⁻²) | n | ICAM-1 average optical density (× 10⁻²) | n | CD68 average optical density (× 10⁻²) |
|---|---|---|---|---|---|---|
| Model Control | 8 | 1.78±1.35 | 8 | 3.96±1.68 | 8 | 1.84±1.25 |
| Statin Treatment | 7 | 1.09±0.54 | 7 | 2.04±1.82* | 7 | 1.68±1.22 |
| Sample A Low Dosage | 7 | 1.41±0.34 | 8 | 2.11±1.53* | 8 | 2.78±2.01 |
| Sample A Medium Dosage | 8 | 0.77±0.46* | 8 | 2.31±1.16* | 7 | 1.39±0.67 |
| Sample A High Dosage | 8 | 0.49±0.51* | 8 | 1.75±1.46* | 8 | 1.86±1.60 |
| Sample B Low Dosage | 8 | 1.24±0.99 | 6 | 2.44±1.19 | 8 | 1.85±1.51 |
| Sample B Medium Dosage | 8 | 1.54±1.11 | 8 | 1.31±1.40* | 8 | 2.22±0.99 |
| Sample B High Dosage | 8 | 1.69±0.85 | 8 | 3.98±1.64 | 7 | 2.83±1.40 |

| | | | | | | |
|---|---|---|---|---|---|---|
| Note: The sample A and B groups were separately compared to the model control group by one-way analysis of variance. Sample A was analyzed for VCAM-1 after SQRT variable conversion. * means P<0.05 compared with the model control group. | | | | | | |

Conclusion: In this experiment, the animals were given drug during the process of establishing the arteriosclerosis model, which corresponds to preventive treatment in the clinic. Under these conditions, TC and LDL-C decreased in female mice at low and high doses of Nib 1, and TG decreased in mice at high doses of sample B. These compounds can regulate blood lipid levels to varying degrees and have a blood lipid improvement effect. The pathological results showed that the medium doses of Nib1 and Nib2 could delay the formation of arterial plaque in ApoE knockout mice to a certain extent, and also showed reduced expression of VCAM-1 and ICAM-1 in the aortic wall.

### Example 2. Evaluation of the efficacy of Nib1, Nib2, X7, and X8 in the treatment of atherosclerosis model induced by high-fat diet in ApoE knockout mice

Study of the effects of test samples Nib1, Nib2, X7, and X8 on the formation of atherosclerosis in ApoE knockout mice.

Reagents: Nib1 was purchased from Sigma (Lot # P1793), and Nib2 from EMD Millipore Corporation (Lot # 573108). Atorvastatin calcium tablets were produced by Beijing Jialin Pharmaceutical Co., Ltd., Lot # MC16035. Sodium carboxymethyl cellulose (CMC-Na) was purchased from Tianjin Fuchen Chemical Reagent Factory (Lot # 20170220); DMSO was purchased from Guangdong Guanghua Technology Co., Ltd. (Lot # 20170215); polyethylene glycol PEG300 was purchased from Shanghai Macklin Biochemical Technology Co., Ltd. (Lot # C10113353).

Methods: A total of 130 SPF grade C57BL/6 ApoE -/- model male mice aged 8 weeks were purchased from Charles River Laboratories (Beijing, China) (animal license number: SCXK (Beijing) 2016-0001, animal qualification certificate No.: 11400700331337). After one week of acclimatization in a SPF-grade animal facility, the animals with less weights were discarded and the rest was randomly divided into 11 groups, with 8 animals in each group. Except for the healthy control group, the rest were fed with 10% high-fat food (the first day of the high-fat diet was recorded as D1). Starting from the 8th week of the high-fat diet, each group of animals was orally given solvents or corresponding drugs, and the volume of administration was 10 ml/kg, once a day for 8 weeks. Animals were observed and administered by gavage once a day and the body weight were measured once a week for 56 days. Atorvastatin calcium tablets were ground and prepared with physiological saline solution. The store solution of the test samples was dissolved in dimethyl sulfoxide solution and prepared with 30% PEG300.

Animals were grouped according to the table below.

**Table 11. Dosages and Groups**

| Group No. | Groups | Doses (mg/kg) | Number | Diet | Dosing Regimen | Duration (weeks) |
|---|---|---|---|---|---|---|
| G1 | healthy control | -- | n=8 | normal | PO, QD | 8 |
| G2 | solvent control | -- | n=8 | high-fat | PO, QD | 8 |
| G3 | atorvastatin | 20 | n=8 | high-fat | PO, QD | 8 |
| G4 | N1 (Nib1) | low 5mpk | n=8 | high-fat | PO, QD | 8 |
| G5 | N1 (Nib1) | high 10mpk | n=8 | high-fat | PO, QD | 8 |
| G6 | N2 (Nib2) | low 2mpk | n=8 | high-fat | PO, QD | 8 |
| G7 | N2 (Nib2) | high 5mpk | n=8 | high-fat | PO, QD | 8 |
| G8 | X7 | low 5mpk | n=8 | high-fat | PO, QD | 8 |
| G9 | X7 | high 10mpk | n=8 | high-fat | PO, QD | 8 |
| G10 | X8 | low 5mpk | n=8 | high-fat | PO, QD | 8 |
| G11 | X8 | high 10mpk | n=8 | high-fat | PO, QD | 8 |

At the end of the experiment, the animals were euthanized by inhalation of carbon dioxide. Blood was taken from the heart, and centrifuged at 3000 r/min at a low temperature centrifuge for 10 min to separate the serum. The levels of total cholesterol TC, triglyceride TG, high-density lipoprotein HDL-C, and low-density lipoprotein LDL-C were measured, and the remaining serum was stored in a refrigerator at -80°C.

Immediately after blood was taken from the heart, it was perfused through the left ventricle with 20ml of normal saline. After bloodletting, the heart and aorta (from the aortic arch to the iliac artery) were isolated. The isolated artery was cut longitudinally from the aortic arch to the iliac artery, fixed in 4% paraformaldehyde for 30 minutes, synchronized with 60% isopropanol for 10 minutes, stained in 60% oil red for 30 minutes, and washed by 60% isopropanol 3 times, 5 minutes each time. Finally, the colored aorta was washed with deionized water and photographed. The proportion of the red plaque area was measured by Image Pro Plus 6.0 (Media Cybernetics, MD, US). The heart samples were fixed in 4% paraformaldehyde solution overnight and then placed in sucrose solution for dehydration. Then, it was embedded with OCT (Sakura, Japan) under a stereo microscope and made into 7µm frozen sections. After HE staining, the slices were used for routine pathological examination. The outflow tract images were collected under an optical microscope at 4* magnification, and 4*, 10*, 20* and 40* magnification eyepieces were used for high-quality morphological observation. The detection standard referred to the histological classification standard of human atherosclerosis disease.

### Results

### Results of Body Weight Changes

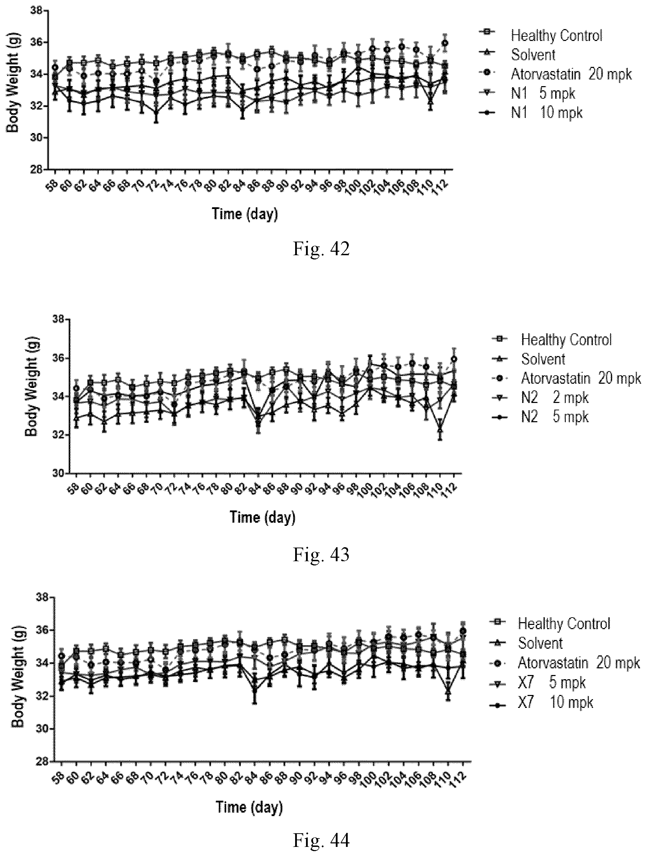

After 8 weeks of high-fat feeding, the animals received different treatments, and their body weight changes were measured daily. The change curves of each group are shown in Figures 42 to 45. The changes in body weight of animals in each group showed similar trends without significant differences, indicating that the toxic and side effects were relatively small.

Figure 42 is a curve of body weight change in compound Nib 1 treatment group, compared with the normal control (Normal), vehicle (Vehicle) and atorvastatin (Atovastatin) treatment groups.

Figure 43 is a curve of body weight change in compound Nib2 (N2) treatment group, compared with the healthy control (Normal), vehicle (Vehicle) and atorvastatin (Atovastatin) treatment groups.

Figure 44 is a curve of body weight change in compound X7 treatment group, compared with the healthy control (Normal), vehicle (Vehicle) and atorvastatin (Atovastatin) treatment groups.

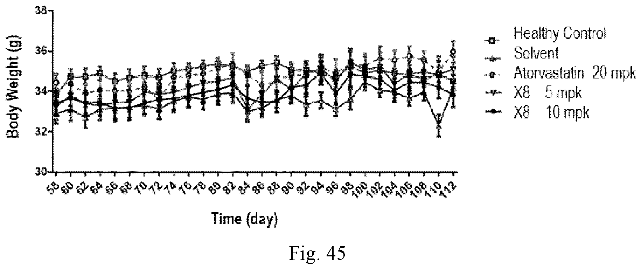

Figure 45 is a curve of body weight change in compound X8 treatment group, compared with the healthy control (Normal), vehicle (Vehicle) and atorvastatin (Atovastatin) treatment groups.

Evaluate the effect of different therapeutic drugs by the ratio of the area of atherosclerotic plaque to the total area of the inner wall of the aortic vessel (En-Face)

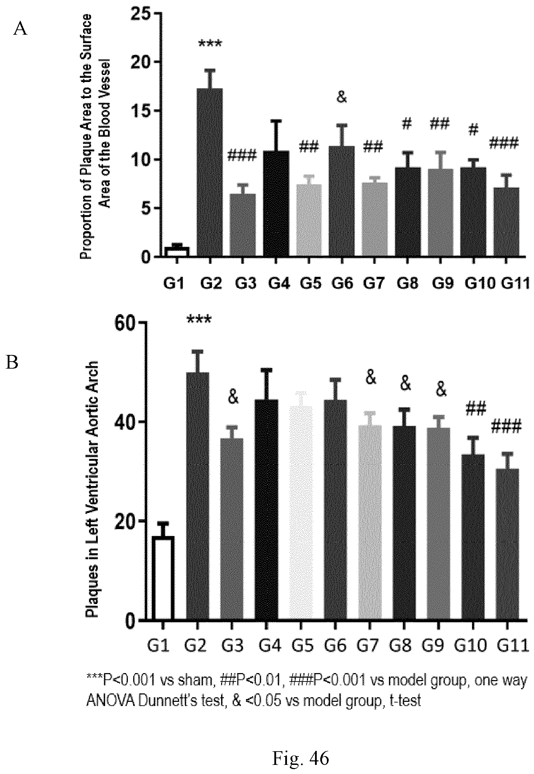

We adopted the widely accepted standard for the severity of arteriosclerosis in animal models, and analyzed the area of all plaques on the inner wall of the aorta. The results are shown in Figures 46 to 52. Compared with the normal control group, the proportion of plaque area in the model group was significantly higher, with an average value of 17.12% (p<0.001), indicating that the animal model was successfully established and the expected plaque formation was achieved. The positive drug atorvastatin treatment group had a significant reduction in plaque formation, only at 6.26% (p<0.001). Compared with the vehicle group, each treatment group has achieved significant curative effects, except for the Nib1 low dosage group. The X8 high dosage group had the optimal therapeutic effect, which was close to the positive drug, with a value of 6.93% (p<0.001). The high dosage treatment groups of Nib1, Nib2, and X7 all achieved good results (p<0.01). Figure 46 is a histogram of the ratios of plaque area on the inner wall of the aorta in each treatment group. The values in Figure 46 are the ratios of plaques to the total area of the inner wall in each group, and the error is standard error ± SEM. The statistical significance analysis is as follows: ***P<0.001 compared with the normal control group; #P<0.05 compared with the vehicle group; ##P<0.01, ###P<0.001, compared with the vehicle group through one-way ANOVA Dunnett's test; & represents P<0.05, compared with the vehicle group by t test.

**Table 12. Plaque Area Ratios**

| | **Groups** | **Mean of Plaque Ratio** | **SD** |
|---|---|---|---|
| G1 | Normal control | 0.84 | 1.16 |
| G2 | Vehicle | 17.12 | 5.31 |
| G3 | Atorvastatin 20mpk | 6.26 | 3.02 |
| G4 | Nib1low dosage, N1 5mpk | 10.74 | 8.53 |
| G5 | Nib1 high dosage, N1 10mpk | 7.33 | 2.59 |
| G6 | Nib2 low dosage, N2 2mpk | 11.22 | 6.07 |
| G7 | Nib2 high dosage, N2 5mpk | 7.43 | 1.90 |
| G8 | X7 low dosage, X7 5mpk | 9.00 | 4.50 |
| G9 | X7 high dosage, X7 10mpk | 8.89 | 4.87 |
| G10 | X8 low dosage, X8 5mpk | 9.02 | 2.54 |
| G11 | X8 high dosage, X8 10mpk | 6.93 | 3.95 |

### Results of lipid metabolism

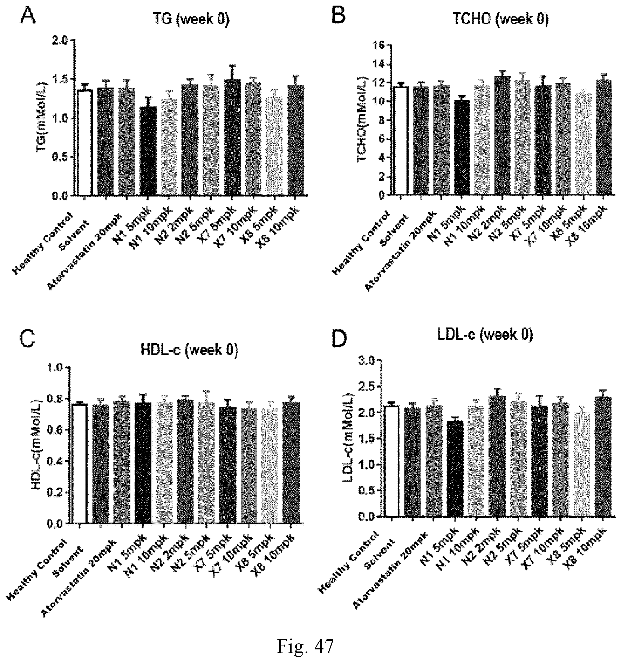

Before the start of the experiment, animal blood samples were analyzed for blood lipid metabolism, including conventional triglyceride (TG), total cholesterol (TCHO), high-density lipoprotein (HDL-c) and low-density lipoprotein (LDL-c). After high-fat or ordinary food feeding for 8 weeks, the blood lipid levels were measured again. After the drug treatment was started, blood lipid measurements were performed every four weeks until the end of the experiment. It can be seen from the results in Figure 47 that compared with the vehicle group, atorvastatin treatment can increase the levels of triglycerides and high-density lipoproteins, and reduce the levels of total cholesterol and low-density lipoproteins. Nib1(N1), Nib2(N2), X7, X8 compound treatment can significantly reduce triglyceride, total cholesterol and low-density lipoprotein levels. Nib1 can significantly reduce the level of high-density lipoprotein, while the other three drugs, Nib2, X7, X8, have no obvious effect. It can be seen that these compounds have varying degrees of regulation on blood lipid levels.

Figure 47 shows the blood lipid levels of each group of animals before the start of the experiment. The values in Figure 47 are the average values of different blood lipids in each group before the start of the experiment, and the error is standard error ± SEM. The statistical significance analysis is as follows: ***P<0.001 compared with the normal control group; #P<0.05, ##P<0.01, ###P<0.001, compared with the vehicle group through one-way ANOVA Dunnett's test; & represents P<0.05, compared with the vehicle group by t test.

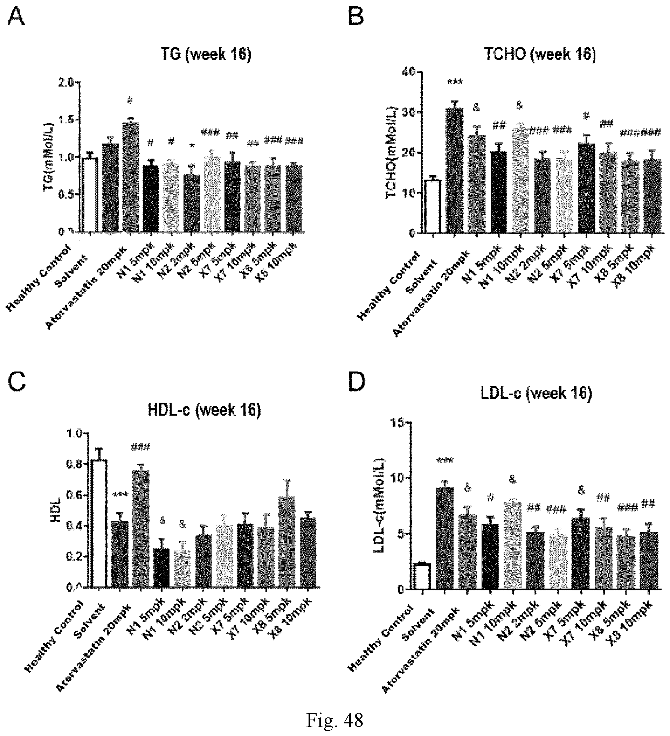

Figure 48 shows the blood lipid levels of the animals at the end of the experiment. The values in Figure 48 are the average values of different blood lipids in each group at the end of the experiment, and the error is standard error ± SEM. The statistical significance analysis is as follows: ***P<0.001 compared with the normal control group; #P<0.05, ##P<0.01, ###P<0.001, compared with the vehicle group through one-way ANOVA Dunnett's test; & represents P<0.05, compared with the vehicle group by t test.

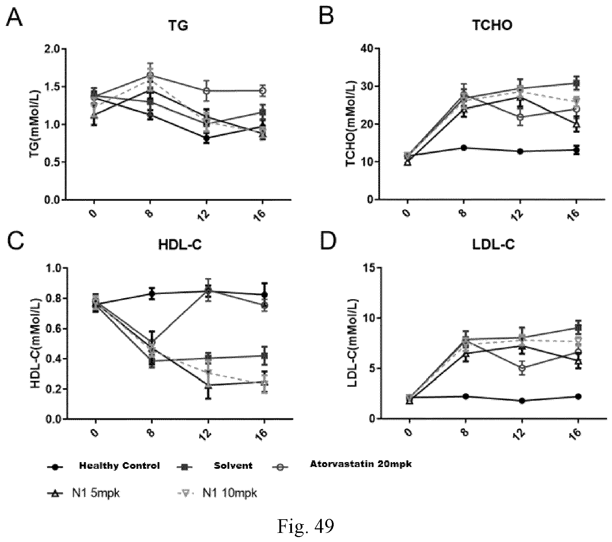

Figure 49 shows the effect of Nib1 treatment on blood lipids. The abscissa axis in Figure 49 is time (weeks), compared to normal control, vehicle group (Vehicle), and atorvastatin treatment group (Atorvastatin 20mpk).

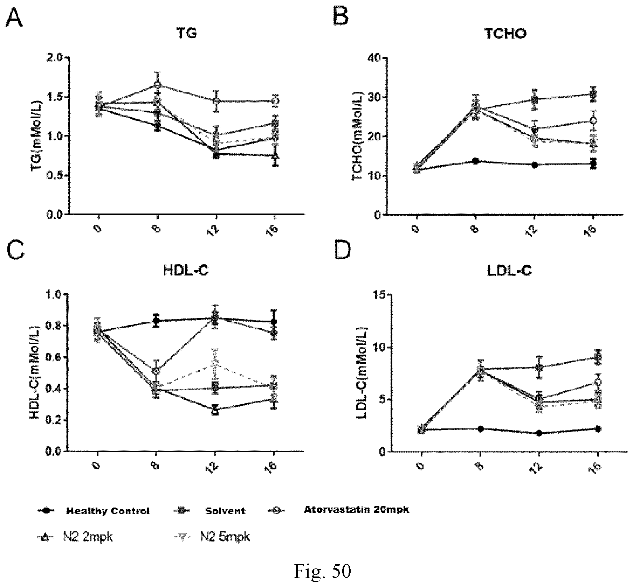

Figure 50 shows the effect of Nib2 treatment on blood lipids. The abscissa axis in Figure 50 is time (weeks), compared to normal control, vehicle group (Vehicle), and atorvastatin treatment group (Atorvastatin 20mpk).

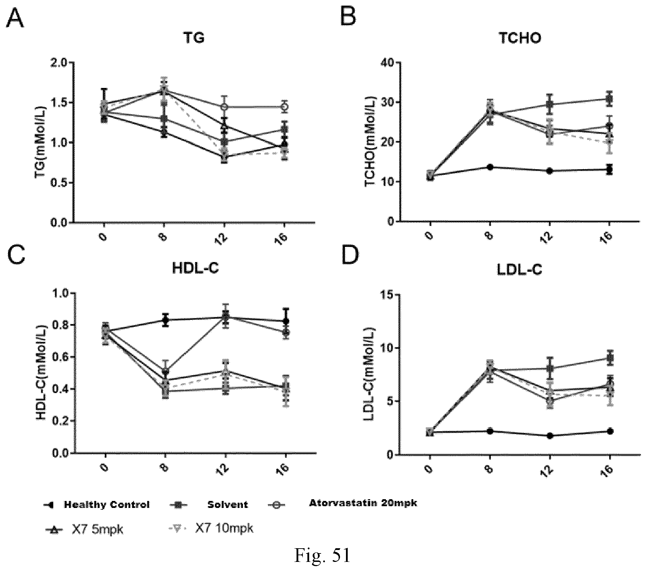

Figure 51 shows the effect of X7 treatment on blood lipids. The abscissa axis in Figure 51 is time (weeks), compared to the normal control, vehicle group (Vehicle), and atorvastatin treatment group (Atorvastatin 20mpk).

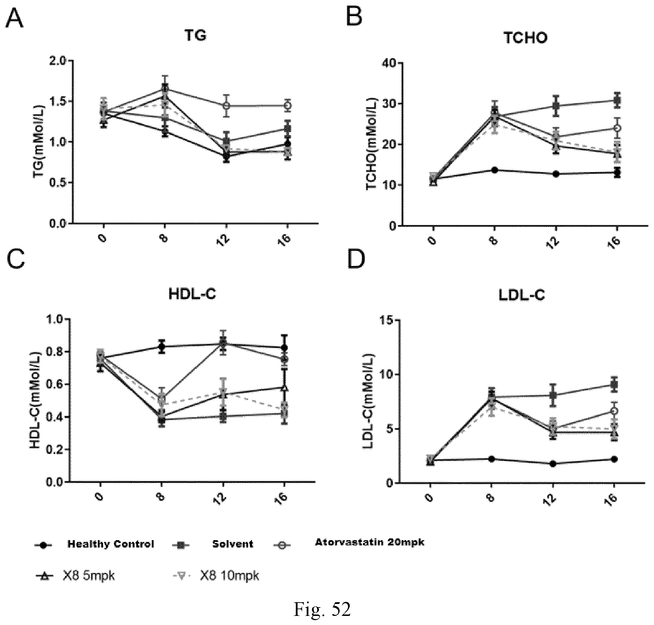

Figure 52 shows the effect of X8 treatment on blood lipids. The abscissa axis in Figure 52 is time (weeks), compared to normal control, vehicle group (Vehicle), and atorvastatin treatment group (Atorvastatin 20mpk).

Conclusion: This experiment proved that different doses of Nib1, Nib2, X7, X8 can significantly alleviate the symptoms of atherosclerosis under therapeutic intervention as mainly indicated by the plaque area ratio. They also had different degrees of regulation on blood lipid levels.

### Example 3. Synthesis of 1-(1-(2-(p-tolyl)acetyl)piperidin-4-yl)-1H-benzo[d]imidazole-2(3H)-one (X7)

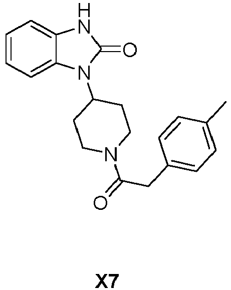

The steps of synthesis are as follows.

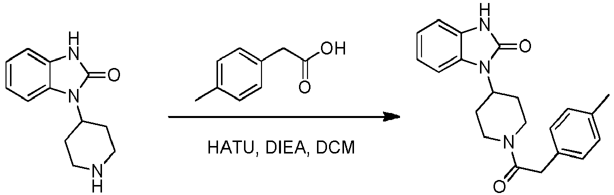

To DCM (20ml) was added 1-(piperidin-4-yl)-1H-benzo[d]imidazole-2(3H)-one hydrochloride (1.50 g, 5.90 mmol), 2-(p-tolyl) acetic acid (1.34 g, 8.90 mmol) and HATU (6.76 g, 17.8 mmol), and DIEA(3.81 g, 29.5 mmol) was added dropwise at 0°C. The reaction mixture was concentrated. The product was purified by high performance liquid chromatography (10-95% CH₃CN aqueous solution) to obtain a white solid (1.16 g, yield 56%). ¹H NMR (400 MHz, CDCl₃): δ 10.83 (s, 1H), 7.21-7.15 (m, 4H), 6.96-6.90 (m, 4H), 4.57 (d, *J=* 13.2 Hz, 1 H), 4.45-4.32 (m, 1 H), 4.07 (d, *J=* 14.0 Hz, 1 H), 3.82-3.67 (m, 2 H), 3.18-3.07 (m, 1 H), 2.72-2.62 (m, 1 H), 2.27 (s, 3 H), 2.08-1.96 (m, 1 H), 1.95-1.80 (m, 1 H), 1.66 (d, *J* = 10.8 Hz, 1 H), 1.57 (d, *J* = 10.8 Hz, 1 H). MS (ESI) *m*/*z* 350.2 [M + H]⁺, purity 96.8% @ 254 nm, 99.6% @ 214 nm.

### Example 4. Synthesis of 1-(1-(2-(4-(trifluoromethyl)phenyl)acetyl) piperidin-4-yl)-1H-benzo[d]imidazole-2(3H)-one (X8)

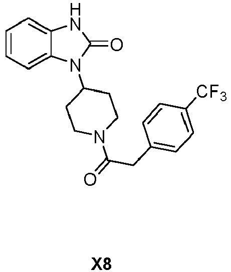

The steps of synthesis are as follows.

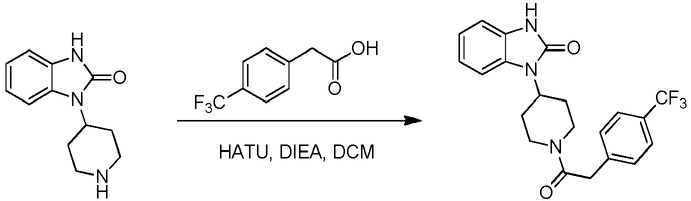

To DCM (50 mL) was added 1-(piperidin-4-yl)-1H-benzo[d]imidazole-2(3H)-one hydrochloride (1.50 g, 5.90 mmol), 2-(4- (trifluoromethyl)phenyl) acetic acid (1.45 g, 7.09 mmol) and HATU (3.37 g, 8.87 mmol), and DIEA (3.81 g, 29.6 mmol) was added. The reaction mixture was stirred at room temperature under N₂ for 3h. The reaction mixture was diluted with DCM (100ml) and washed with NH₄Cl aqueous solution (100ml). The organic layer was isolated, dried over Na₂SO4, and concentrated under reduced pressure to obtain a crude product, which was purified by high performance liquid chromatography (10-50% CH₃CN aqueous solution) to obtain a white solid (1.75 g, yield 74%). ¹H NMR (400 MHz, CDCl₃): δ10.84 (s, 1 H), 7.71 (d, *J=* 8.0 Hz, 2 H), 7.53(d, *J=* 8.0 Hz, 2 H), 7.09-7.03 (m, 1 H), 6.98-6.92 (m, 3H), 4.57 (d, *J* = 13.2 Hz, 1 H), 4.45-4.32 (m, 1 H), 4.13 (d, *J* = 14.0 Hz, 1 H), 3.98-3.85 (m, 2 H), 3.19 (t, *J* = 12.0 Hz, 1 H), 2.71 (t, *J* = 12.0 Hz, 1 H), 2.16-2.01 (m, 2 H), 1.68 (m, t, *J* = 12.0 Hz, 2 H). MS (ESI) *m*/*z* 404.1 [M + H]⁺, purity 92.4% @ 254 nm, 99.7% @ 214 nm.

The raw materials and equipment used in the present invention, unless otherwise specified, are all commonly used raw materials and equipment in the field; the methods used in the present invention, unless otherwise specified, are all conventional methods in the field.

The above are only preferred embodiments of the present invention and do not limit the present invention in any way. It is understood that simple modification, alteration and equivalent transformation of the above embodiments are still within the scope of the invention without departing from the technical essence of the present invention.

## Claims

1. Use of a compound in the preparation of a medicament for prevention and/or treatment of atherosclerosis, wherein the compound is selected from the group consisting of a compound of formula I, Nib2 and a pharmaceutically acceptable salt thereof; 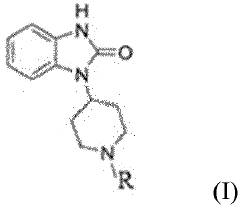
wherein, R is 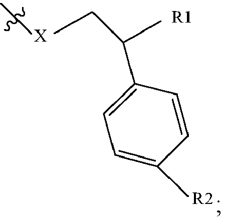
wherein, X is -CH2-CH2- or 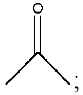
R1 is H or 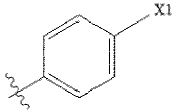 and X1 is a halogen;
R2 is CH3 or CX2, and X2 is a halogen;
wherein, Nib2 has a chemical formula of C₁₆H₁₁N₃O₃ and a structural formula of

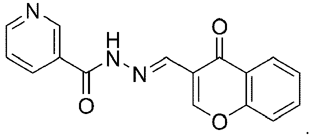

2. The use of claim 1, wherein the compound of formula I is Nib1, X7 or X8, wherein Nib1 has a chemical formula of C₂₈H₂₉F₂N₃O and a structural formula of 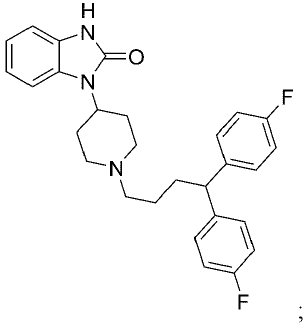
X7 has a chemical formula of C₂₁H₂₃N₃O₂ and a structural formula of 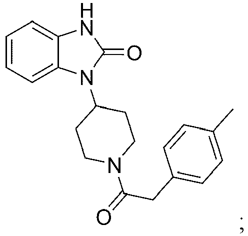 and
X8 has a chemical formula of C₂₁H₂₀F₃N₃O₂ and a structural formula of

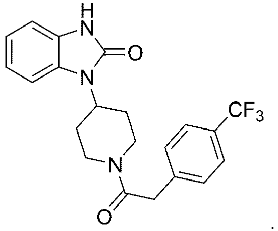

3. The use of claim 2, wherein the Nib1 is administered at a dosage of 0.1 to 1.098 mg/kg; and/or, the Nib2 is administered at a dosage of 0.109 to 5 mg/kg; and/or, the X7 and/or X8 is administered at a dosage of 0.1 to 5 mg/kg; and
preferably, the Nib1 is administered at a dosage of 0.219 to 1.098 mg/kg, such as 0.549 mg/kg; and/or, the Nib2 is administered at a dosage of 0.109 to 0.549 mg/kg, such as 0.219 mg/kg; and/or, the X7 and/or X8 is administered at a dosage of 0.549 to 1.098 mg/kg.

4. The use of any of claims 1 to 3, wherein the compound is formulated into a pharmaceutical composition, wherein the pharmaceutical composition preferably comprises a pharmaceutically acceptable carrier and/or excipient;
and/or, the compound is formulated into an oral or injection formulation, and the oral formulation is preferably a capsule or a tablet.

5. The use of any of claims 1 to 4, wherein the compound can improve abnormal blood lipid metabolism, preferably reduce the levels of TC, HDL and/or LDL, significantly inhibit the foaming of macrophages, significantly reduce the deposition of macrophages in the atheroma, reduce the deposition of lipidic necrosis substances and/or decrease the formation of atherosclerotic plaques.

6. A drug for treating atherosclerosis, comprising a compound selected from a group consisting of a compound of formula I, Nib2 and a pharmaceutically acceptable salt thereof; 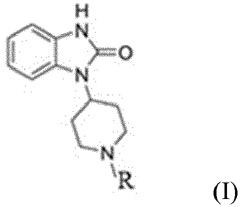
wherein, R is 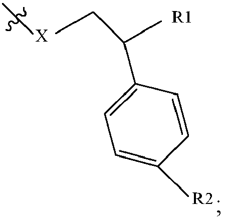
wherein, X is -CH2-CH2- or 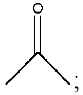
R1 is H or 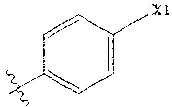 and X1 is a halogen;
R2 is CH3 or CX2, and X2 is a halogen;
wherein, Nib2 has a chemical formula of C₁₆H₁₁N₃O₃ and a structural formula of 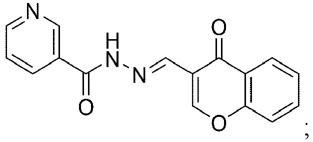
preferably, the compound of formula I is Nib1, X7 or X8, wherein Nib1 has a chemical formula of C₂₈H₂₉F₂N₃O and a structural formula of 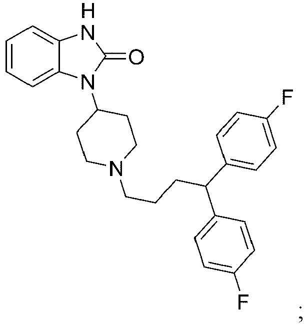
X7 has a chemical formula of C₂₁H₂₃N₃O₂ and a structural formula of 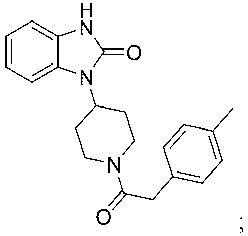 and
X8 has a chemical formula of C₂₁H₂₀F₃N₃O₂ and a structural formula of

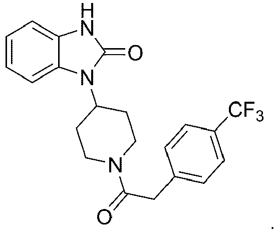

7. The drug of claim 6, wherein the Nib1 is administered at a dosage of 0.1 to 1.098 mg/kg; and/or, the Nib2 is administered at a dosage of 0.109 to 5 mg/kg; and/or, the X7 and/or X8 is administered at a dosage of 0.1 to 5 mg/kg; and
preferably, the Nib1 is administered at a dosage of 0.219 to 1.098 mg/kg, such as 0.549 mg/kg; and/or, the Nib2 is administered at a dosage of 0.109 to 0.549 mg/kg, such as 0.219 mg/kg; and/or, the X7 and/or X8 is administered at a dosage of 0.549 to 1.098 mg/kg.

8. The drug of claim 6 or 7, wherein the drug further comprises a pharmaceutically acceptable carrier and/or excipient;
and/or, the drug is formulated into a pharmaceutical composition,
and/or, the compound is formulated into an oral or injection formulation; and the oral formulation is preferably a capsule or a tablet.

9. The drug of any of claims 6 to 8, wherein the drug can improve abnormal blood lipid metabolism, preferably reduce the levels of TC, HDL and/or LDL, significantly inhibit the foaming of macrophages, significantly reduce the deposition of macrophages in the atheroma, reduce the deposition of lipidic necrosis substances and/or decrease the formation of atherosclerotic plaques.

10. A compound of any of the preceding claims for use in prevention and/or treatment of atherosclerosis.

11. A method for preventing and/or treating atherosclerosis, comprising using a compound of any of the preceding claims for treatment.

12. A drug for improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques, wherein the drug comprises a compound of any of the preceding claims.

13. A method for improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques, wherein the method comprises using a compound of any of the preceding claims for treatment.

14. A compound of any of the preceding claims for use in improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques.

15. Use of a compound of any of the preceding claims in the preparation of a medicament for improving abnormal blood lipid metabolism, preferably reducing the levels of TC, HDL and/or LDL, significantly inhibiting the foaming of macrophages, significantly reducing the deposition of macrophages in the atheroma, reducing the deposition of lipidic necrosis substances and/or decreasing the formation of atherosclerotic plaques.
